# EUROPÄISCHE PATENTSCHRIFT

(11) **EP 2 096 102 B1**
(45) Veröffentlichungstag und Bekanntmachung des Hinweises auf die Patenterteilung: **26.03.2014**
(21) Anmeldenummer: 09002178.3
(22) Anmeldetag: 17.02.2009
(51) Int. Cl.: C07C 209/78, C07C 211/50, C07C 263/10, C07C 265/14, C25B 15/08, C25B 1/26

(54) **Verfahren zur Herstellung von Methylen-diphenyl-diisocyanaten**
Method for creating methylene-diphenyl-diisocyanates
Procédé de fabrication de méthylène-diphényle-diisocyanates

(30) Priorität: 01.03.2008 DE 102008012037
(43) Veröffentlichungstag der Anmeldung: 02.09.2009
(73) Patentinhaber: Bayer Intellectual Property GmbH, 40789 Monheim (DE)
(72) Erfinder: Pohl, Fritz, Dr., 25541 Brunsbüttel (DE); Bulan, Andreas, 40764 Langenfeld (DE); Weber, Rainer, Dr., 51519 Odenthal (DE); Adamson, Richard, 42799 Leichlingen (DE); Böhm, Matthias, 51373 Leverkusen (DE); Six, Christian, Dr., Baytown, TX 77520-9730 (US)
(74) Vertreter: BIP Patents

(56) Entgegenhaltungen:
- EP-A- 1 743 882
- EP-A- 1 813 598
- WO-A-97/24320

## Beschreibung

Die Erfindung geht aus von einem Verfahren zur Herstellung von Methylen-diphenyl-diisocyanaten (MDI) durch Umsetzung von Anilin mit Formaldehyd in Gegenwart von Salzsäure zu einem Gemisch von Di- und Polyaminen der Diphenylmethanreihe und Umsetzung des erhaltenen Gemischs der Di- und Polyamine der Diphenyl-methanreihe mit Phosgen zu einem Gemisch von Di- und Polyisocyanaten der Diphenylmethanreihe.

Aromatische Diisocyanate sind wichtige und vielseitige Rohstoffe für die Polyurethanchemie. Hierbei sind Toluylendiisocyanat (TDI) und MDI die wichtigsten technischen Isocyanate.

Der allgemeine Begriff "MDI" wird im Fachgebiet und im Sinne dieser Erfindung als Oberbegriff für Methylen(diphenyldiisocyanate) und Polymethylen-polyphenylen-polyisocyanate, die Polyisocyanate der Diphenylmethanreihe, verwendet. Unter den Polyisocyanaten der Diphenylmethanreihe werden insbesondere Verbindungen und Verbindungsgemische folgenden Typs verstanden: 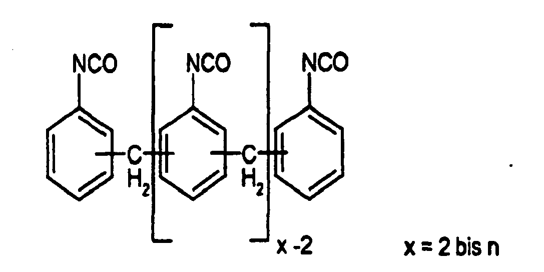 wobei n für eine natürliche Zahl ≥ 2 steht.

Der Begriff Methylen(diphenyldiisocyanat) umfasst die Isomere 2,2'-Methylen(diphenyldiisocyanat) (2,2'-MDI), 2,4'-Methylen(diphenyldiisocyanat) (2,4'-MDI) und 4,4'-Methylen(diphenyldiisocyanat) (4,4'-MDI). Zusammenfassend werden diese Isomere als "Monomer-MDI" bezeichnet. Der Begriff Polymethylen-polyphenylen-polyisocyanate umfasst das sog. "Polymer-MDI" (PMDI), das Monomer-MDI und höhere Homologe des Monomer-MDI enthält.

In allen großtechnisch relevanten Herstellverfahren wird MDI durch Phosgenierung der entsprechenden Polyamine der Diphenylmethanreihe gewonnen. Die MDI-Synthese geschieht dabei üblicherweise in einem zweistufigen Prozess. Zunächst wird Anilin mit Formaldehyd zu einem Gemisch aus oligomeren und isomeren Methylen(diphenyldiaminen) und Polymethylenpolyaminen, dem sogenannten Roh-MDA kondensiert. Dieses Roh-MDA wird anschließend in einem zweiten Schritt mit Phosgen in an sich bekannter Weise zu einem Gemisch der entsprechenden oligomeren und isomeren Methylen(diphenyldiisocyanate) und Polymethylen-polyphenylen-polyisocyanate, dem sogenannten Roh-MDI umgesetzt. Hierbei bleibt die Isomeren- und Oligomerenzusammensetzung unverändert. Meist wird dann ein Teil der 2-Kern Verbindungen in einem weiteren Verfahrensschritt , z.B. durch Destillation oder Kristallisation abgetrennt, wobei Polymer-MDI (PMDI) als Rückstand verbleibt.

Die kontinuierliche, diskontinuierliche oder halbkontinuierliche Herstellung von Polyaminen der Diphenylmethanreihe, im Folgenden auch kurz MDA genannt, ist in zahlreichen Patenten und Publikationen beschrieben (siehe z.B. H.J.Twitchett, Chem.Soc.Rev. 3(2), 209 (1974), M.V. Moore in: Kirk-Othmer Encycl. Chem. Technol., 3rd. Ed., New York,2, 338-348 (1978). Üblicherweise erfolgt die Herstellung von MDA durch Umsetzung von Anilin und Formaldehyd in Anwesenheit saurer Katalysatoren. Üblicherweise wird Salzsäure als saurer Katalysator eingesetzt, wobei der saure Katalysator gemäß dem Stand der Technik zum Ende des Prozesses und vor den abschließenden Aufarbeitungsschritten, beispielsweise der destillativen Entfernung von überschüssigem Anilin, durch Zusatz einer Base, typischerweise wässriger Natronlauge, neutralisiert und somit verbraucht wird. In der Regel erfolgt die Zugabe des Neutralisationsmittels derart, dass sich das erhaltene Neutralisationsgemisch in eine organische, die Polyamine der Diphenylmethanreihe und überschüssiges Anilin enthaltende Phase und eine wässrige Phase, welche neben Natriumchlorid noch Reste organischer Bestandteile enthält, auftrennen lässt. Die wässrige Phase wird in der Regel nach Entfernung der organischen Bestandteile als anorganisch belastetes Abwasser entsorgt.

Es ist bekannt, dass sich die Zusammensetzung des nach dem skizzierten Prozess hergestellten Roh-MDAs, mithin des daraus hergestellten Roh-MDIs, in weitem Rahmen durch die angewandten Reaktionsbedingungen variieren lässt. Von besonderer Bedeutung sind in diesem Zusammenhang das Mol-Verhältnis der zur Umsetzung gebrachten Komponenten Anilin und Formaldehyd (im folgenden auch kurz A/F-Verhältnis genannt) wie auch die zugesetzte Katalysatormenge, üblicherweise als Protonierungsgrad (Mol-Verhältnis von zugesetztem Katalysator zu eingespeistem Anilin) angegeben.

Über hohe A/F-Verhältnisse ist ein Roh-MDA mit einem hohen Monomeranteil zugänglich, hohe Protonierungsgrade fördern dabei die Bildung des technisch als Vorstufe für das entsprechende 4,4'-MDI besonders bedeutsamen 4,4'-MDAs. Beide Maßnahmen führen aber zu einem hohen Salzanfall, der zum einen einen hohen wirtschaftlichen Verlust, zum anderen eine mögliche Belastung der Umwelt beinhaltet.

Es hat nicht an Versuchen gefehlt, Verfahren zu entwickeln, bei welchen das bei der MDA-Herstellung anfallende Abwasser und die über das Abwasser zu entsorgende Salzfracht deutlich verringert oder gänzlich vermieden werden.

DE 2 238 920 A1 offenbart ein Verfahren zur Herstellung von Polyaminen der Diphenylmethanreihe durch Kondensation von Anilin mit Formaldehyd in Gegenwart von Wasser und sauren Katalysatoren, bei dem das ausreagierte wässrige Kondensationsgemisch mit einem hydrophoben Lösungsmittel extrahiert, die so erhaltene Lösungsmittelphase zu den Polyaminen aufgearbeitet und die wässrige Phase nach Zugabe von Anilin und Formaldehyd im Kreislauf an den Prozessanfang zurückgeführt wird. Gemäß der Lehre von DE 2 238 920 A1 können das bei der Umsetzung der Edukte entstehende Kondensationswasser und überschüssiges Wasser, das mit den Ausgangsmaterialien in das System gelangt, an geeigneter Stelle aus dem System ausgeschleust werden. Bei dem Verfahren fällt ein Abwasser an, das im Wesentlichen nur das mit den Edukten eingetragene sowie das bei der Reaktion des Anilins mit dem Formaldehyd entstehende Wasser enthält, der Einsatz von Natronlauge entfällt. Nachteilig an diesem Verfahren ist die Verwendung eines zusätzlichen organischen Lösungsmittels, dessen Einsatz in einer Extraktion sowie die Abtrennung durch Destillation apparativ und energetisch aufwändig sind. Zusätzlich sind große Volumina wässriger Phase im Prozesskreislauf zu führen und ebenfalls destillativ aufzubereiten.

Die Reduktion der aufzubereitenden wässrigen Phasen bei gleichzeitiger Rückführung des eingesetzten homogenen Katalysators ist Gegenstand von DE 2 426 116 A1. Nach der Lehre von DE 2 426 116 A1 ist es besonders vorteilhaft, die Umsetzung von Anilin mit Formaldehyd mehrstufig durchzuführen, dabei Anilin und Formaldehyd in einer ersten Stufe in Abwesenheit eines Katalysators zu einem so genannten Vorkondensat ("Aminal") umzusetzen, nach beendeter Umsetzung das Wasser abzutrennen, dann das Vorkondensat in Gegenwart von sauren Salzen des Anilins oder seiner Kondensationsprodukte mit dem Formaldehyd in Anwesenheit von polaren, organischen Lösungsmitteln, die unter den Reaktionsbedingungen indifferent bleiben, umzusetzen und abschließend den eingesetzten Katalysator mit Wasser aus dem Reaktionsgemisch auszuwaschen und auf die Umsetzungsstufe des Vorkondensats zurückzuführen. Nachteilig sind auch hier der Einsatz eines zusätzlichen organischen Lösungsmittels und der hierdurch bedingte hohe apparative und energetische Aufwand für Extraktion und Destillation.

Die intermediäre Bildung eines Vorkondensats offenbart auch DE 2 623 681 A1, zusätzlich den Einsatz eines heterogenen sauren Katalysators auf Basis von Ton, Zeoliten oder Diatomeenerden für die Umsetzung der Vorkondensate zu den Polyaminen der Diphenylmethanreihe, wobei nach der Lehre von DE 2 623 681 A1 der Katalysator nach beendeter Umsetzung durch Filtration oder Zentrifugieren von dem Polyamingemisch abgetrennt und auf die Reaktionsstufe des Vorkondensats zurückgeführt werden kann. Für die Umlagerungsreaktion muss das Aminal jedoch aufwändig vorgetrocknet werden, ein weiterer Nachteil des Verfahrens ist die Anreicherung von Reaktionsprodukten auf dem Katalysator, was in der Regel eine unzureichende Standzeit des Katalysators zur Folge hat.

Die Umwandlung des Vorkondensats aus Anilin und Formaldehyd in ein polymerarmes Zweikern-MDA unter Einsatz von festen sauren Katalysatoren durchzuführen und dabei die Vorteile der Katalyse mit Feststoffen bei für technische Verfahren geforderten, ausreichend langen KatalysatorStandzeiten zu nutzen, ohne dabei nennenswerte Restmengen an polymeren MDA-Basen oder nicht vollständig umgelagerter Intermediate ("Aminobenzylaniline") im Endprodukt zu erhalten ist ebenfalls Gegenstand der Lehre von EP 1 257 522 B1. Nachteilig an dem in EP 1 257 522 B1 offenbarten Verfahren sind aber ebenfalls die notwendige aufwendige Trocknung des Vorkondensats sowie die Anreicherung von Reaktionsprodukten auf den eingesetzten Katalysatoren, deren dadurch bedingte geringe Standzeiten.

Auch US 7 105 700 B2 offenbart den Einsatz heterogener Katalysatoren bei der Umlagerung der Aminale, der primären Reaktionsprodukte des Anilins mit dem zugesetzten Formaldehyd, wobei als Katalysatoren oberflächenmodifizierte Zeolite Verwendung finden. Nach der Lehre von US 7 105 700 B2 ist zunächst jedoch ebenfalls eine aufwendige Vortrocknung der Aminale notwendig, und wird die für einen technischen Maßstab notwendige Standzeit der eingesetzten Katalysatoren durch die Verwendung eines zusätzlichen Lösungsmittels erreicht, das nach erfolgter Umsetzung destillativ zu entfernen ist, was ebenfalls nachteilig ist.

Die Vorteile einer homogenen Katalyse bei gleichzeitiger Rückgewinnung des eingesetzten sauren Katalysators in fester Form offenbart WO 2005/007 613 A1. WO 2005/007 613 A1 offenbart ein Verfahren zur Herstellung von Polyaminen der Diphenylmethanreihe durch Umsetzung von Anilin mit einem Methylengruppen liefernden Agens, bei welchem die als Katalysator eingesetzte Säure nach Beendigung der Umlagerungsreaktion mittels Adsorption, z.B. an einem Ionenaustauscher, aus dem Reaktionsgemisch entfernt und anschließend durch Regeneration des Adsorptionsmittels zurück gewonnen wird. Nachteilig an diesem Verfahren ist der hohe Aufwand zur vollständigen Ad- und Desorption des eingesetzten Katalysators. Zur vollständigen Entfernung des Katalysators aus dem Reaktionsgemisch offenbart WO 2005/007 613 A1 deshalb eine nachgehende Neutralisation mit üblichen basischen Neutralisationsmitteln, um letzte Spuren der Säure zu entfernen, so dass auch dieses bekannte Verfahren einen hohen technischen Aufwand erfordert.

Zusammenfassend ist festzuhalten, dass bei den aus dem Stand der Technik bekannten Verfahren unter Minimierung der mit dem Abwasser abgegebenen Salzfrachten der Aufwand für die MDA-Herstellung größer wird. Bei Verfahren mit Einsatz heterogenen Katalysatoren bereiten die Standzeit und Regenerierung der Katalysatoren in der Regel große Probleme, bei Verfahren unter Einsatz homogener Katalysatoren benötigen die Extraktionsverfahren den Einsatz zusätzlicher Stoffe und Apparate. Standardverfahren unter Einsatz nur geringer Mengen homogenen Katalysators liefern nicht die Isomeren-Zusammensetzung in der gewünschten Bandbreite, es sind dann apparativ und energetisch aufwendige Nachbereitungen erforderlich.

Aufgabe der Erfindung ist es, ein integriertes Verfahren für die Herstellung von MDI zu finden, bei welchem das bei der Herstellung der Vorstufe MDA anfallende Abwasser und die über dieses Abwasser außerhalb des integrierten Verfahrens zu entsorgende Salzfracht deutlich verringert oder gänzlich vermieden werden, ohne dass der Aufwand der MDI-Herstellung steigt, ohne dass Ausbeuten, Isomeren- und Homologenverhältnisse und deren Steuerung sich verschlechtern und ohne dass die Qualität des erhaltenen MDI negativ beeinflusst wird.

Die Aufgabe konnte durch das erfindungsgemäße Verfahren gelöst werden. Gegenstand der Erfindung ist ein Verfahren zur Herstellung von Methylen-diphenyl-diisocyanaten, wenigstens umfassend die Schritte
A1) Umsetzung von Anilin mit Formaldehyd in Gegenwart von Salzsäure zu einem Reaktionsgemisch enthaltend Di- und Polyamine der Diphenylmethanreihe ("MDA"), gegebenenfalls zumindest teilweise unter Entfernung des mit den Edukten eingetragenen oder bei der Umsetzung entstehenden Wassers aus dem Reaktionsgemisch,
A2) Neutralisation des gemäß A1) gebildeten Reaktionsgemisches unter Einsatz wässriger Alkalihydroxidlösungen unter Erhalt einer organischen Phase enthaltend das Gemisch der Di- und Polyamine der Diphenylmethanreihe und einer wässrigen Alkalichlorid-haltigen Lösung,
A3) Abtrennung der in Schritt A2) erhaltenen organischen Phase von der wässrigen Phase und Aufbereitung der organischen Phase unter Gewinnung des Gemischs der Di- und Polyamine der Diphenylmethanreihe (MDA),
A4) Einstellung der gemäß Schritt A3) verbliebenen, Alkalichlorid-haltigen Lösung auf einen pH-Wert < 8, Reinigung der Alkalichlorid-haltigen Lösung so, dass diese auf einen TOC-Wert < 200 ppm, bevorzugt < 50 ppm, ganz besonders bevorzugt < 20 ppm gebracht wird, wobei die Alkalichlorid-haltige Lösung ggf. zumindest teilweise mit den in Schritt A1) aus dem Reaktionsgemisch entfernten und /oder mit den bei der Aufarbeitung der organischen Phase gemäß Schritt A3) anfallenden wässrigen Phasen vereinigt wird,
B) Umsetzung des in Schritt A3) erhaltenen Gemischs der Di- und Polyamine der Diphenylmethanreihe mit Phosgen zu einem Gemisch der D- und Polyisocyanate der Diphenylmethanreihe (MDI) und Chlorwasserstoff.
   dadurch gekennzeichnet, dass
C) die in Schritt A4) erhaltene gereinigte Alkalichlorid-haltige Lösung zumindest teilweise einer elektrochemischen Oxidation unter Bildung von Chlor, Alkalilauge und ggf. Wasserstoff zugeführt wird, und
D) wenigstens ein Teil des gemäß Schritt C) hergestellten Chlors zur Herstellung des in Schritt B) eingesetzten Phosgens eingesetzt wird.

Methylen-diphenyl-diisocyanate im Sinne der Erfindung sind Methylen(diphenyldiisocyanate) und Polymethylen-polyphenylen-polyisocyanate, die Polyisocyanate der Diphenylmethanreihe. Unter den Polyisocyanaten der Diphenylmethanreihe werden insbesondere Verbindungen und Verbindungsgemische folgenden Typs verstanden: 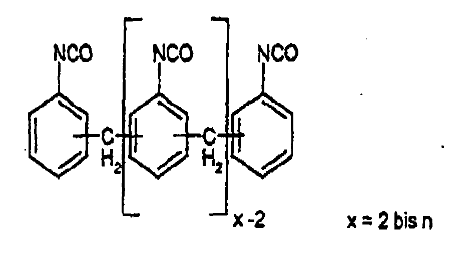 wobei n für eine natürliche Zahl ≥ 2 steht.

Eine weitere bevorzugte Variante des neuen Verfahrens ist **dadurch gekennzeichnet, dass** wenigstens ein Teil der gemäß Schritt C) hergestellten Alkalilauge in die Neutralisation gemäß Schritt A2) zurückgeführt wird.

Die Reinigung der Alkalichlorid-haltigen Lösung in Schritt A4) nach Einstellung der Alkalichlorid-haltigen Lösung auf einen pH-Wert kleiner 8 erfolgt bevorzugt durch Strippen mit Wasserdampf und nachfolgender Behandlung mit Aktivkohle.

Bei dem erfindungsgemäßen integrierten MDI-Verfahren können alle für die MDA-Herstellung bekannten Verfahren unter Einsatz von Chlorwasserstoff-Katalysator als Gas oder in Form von Salzsäure in Schritt A1) bis A3) angewendet werden, bei denen eine Alkalichlorid-haltige wässrige Lösung anfällt. Wesentlich für das neue MDI-Verfahren ist allein, dass die bei der MDA-Herstellung anfallende Alkalichlorid-haltige Lösung wenigstens zum Teil nach Aufbereitung in Schrittt A4) einer Alkalichloridelektrolyse zugeführt und dort, ggf. nach Wasserentzug oder nach einer entsprechender Aufstockung mit dem entsprechenden Alkalichlorid, elektrochemisch unter Gewinnung von Chlor oxidiert und das entstandene Chlor zumindest teilweise in die Phosgenerzeugung für die Umsetzung der Polyamine zu den entsprechenden Polyisocyanaten der Diphenylmethanreihe zurückgeführt wird.

In einer besonders bevorzugten Ausführung des erfindungsgemäßen Verfahrens erfolgt die MDA-Herstellung gemäß Schritt A1) bis A3) nach dem in EP 1 813 598 A1 offenbarten Verfahren, auf das hier ausdrücklich Bezug genommen wird. EP 1 813 598 A1 offenbart ein Verfahren, bei dem
1) Anilin mit Formaldehyd in Gegenwart eines sauren Katalysators zu einem Reaktionsgemisch enthaltend Di- und Polyamine der Diphenylmethanreihe umgesetzt wird, und
2) das Reaktionsgemisch enthaltend Di- und Polyamine neutralisiert wird, und
3) das neutralisierte Reaktionsgemisch enthaltend Di- und Polyamine in eine organische Phase enthaltend Di- und Polyamine und eine wässrige Phase aufgetrennt wird, und
4) ggf. die organische Phase mit Wasser gewaschen wird, und
5) aus der organischen Phase überschüssiges Anilin destillativ entfernt wird, und
6) die in den Schritten 1) - 5) anfallenden Abwässer und Kondensate teilweise oder vollständig zusammengeführt werden, wobei zumindest die in den Schritten 3) und 5) erhaltenen Prozesswässer und Kondensate jeweils zumindest teilweise zusammen geführt werden, und wobei eine Mischung enthaltend Wasser, Di- und Polyamine, Anilin und Salze des in Schritt 1) eingesetzten Katalysators erhalten wird, und
7) die in Schritt 6) erhaltene Mischung einer Phasentrennung unterzogen wird, wobei ein Anilin enthaltend Di- und Polyamine erhalten wird, und
8) das bei der Phasentrennung erhaltene Anilin enthaltend Di- und Polyamine anschließend zumindest teilweise in die Umsetzung in Schritt 1) zurückgeführt wird.

In einer ganz besonders bevorzugten Form des erfindungsgemäßen Verfahrens wird bei der MDA-Herstellung gemäß EP 1 813 598 in Schritt 6) eine Mischung enthaltend Wasser sowie 0,001 bis 5 Gew.-% MDA, 0,5 bis 60 Gew.-% Anilin und 1- 25 Gew.-% an Salzen des in Schritt 1) eingesetzten sauren Katalysators, jeweils bezogen auf das Gewicht der Mischung, erhalten, wird dann das so erhaltene Prozesswasser in einer Extraktion, bevorzugt mehrstufig, bei einer Temperatur von 30 bis 120 °C, bevorzugt 70 bis 110°C mit Anilin im Gewichtsverhältnis von Anilin zu Prozesswasser von 0,05 bis 1:1, bevorzugt 0,1 bis 0,3:1, extrahiert, durch Zugabe von Salzsäure auf einen pH-Wert von kleiner oder gleich 8 gestellt, abschließend aus dem so extrahierten Prozesswasser Anilin destillativ und /oder durch Strippen mit Wasserdampf entfernt.

Die skizzierte besonders bevorzugte Form des erfindungsgemäßen Verfahrens hat den Vorteil, dass der anfallende Alkalichlorid-haltige Abwasserstrom bereits weitgehend von seinen organischen Bestandteilen befreit worden ist, für seinen Einsatz in einer Alkalichlorid-Elektrolyse kein oder nur noch ein geringer Aufbereitungsaufwand erforderlich ist. Wie oben dargestellt, sind für das erfindungsgemäße MDI-Verfahren jedoch alle MDA-Verfahren geeignet, bei denen eine wässrige Alkalichlorid-Lösung anfällt.

Unabhängig von dem angewandten Verfahren zur MDA-Herstellung sollten die bei der MDA-Herstellung anfallenden wässrigen Salzlösungen vor einem Einsatz in der Alkalichloridelektrolyse von noch enthaltenen organischen Verunreinigungen, insbesondere Anilin, MDA und seinen Vorläufern, Formaldehyd, Methanol und Spuren Phenol befreit werden, wobei Methanol als Verunreinigung des Formaldehyds, Phenol als Verunreinigung des Anilins in den Prozess gelangen kann. Die Reinigung kann zum Beispiel durch Extraktion, durch Strippung mit Dampf, durch Ozonolyse und Austreiben des entstehenden Kohlendioxids, Adsorption an einem Adsorptionsmittel wie Aktivkohle oder durch andere Verfahren oder auch durch Kombination mehrerer Verfahren erfolgen.

Im neuen Verfahren wird die Alkalichlorid-haltige wässrige Lösung vor der Oxidation C) auf einen TOC-Wert < 200 ppm, bevorzugt < 50 ppm, ganz besonders bevorzugt < 20 ppm gebracht.

Die Reinigung der bei den angewandten MDA-Verfahren anfallenden Alkalichlorid-haltigen Lösung kann dabei separat oder - z.B. wie oben dargestellt - zusammen mit anderen Wasserströmen erfolgen. Bevorzugt werden die bei der MDA-Herstellung anfallenden Wasserströme zusammengefasst und gemeinsam gereinigt; vor der Reinigung nach Schritt A4) der resultierenden Lösung wird der pH-Wert der Lösung auf einen Wert kleiner 8 gestellt.

Gemäß einem bevorzugten Verfahren wird die Erniedrigung des pH-Wertes mit Salzsäure oder Chlorwasserstoff durchgeführt. Der grundsätzlich denkbare, aber im erfindungsgemäßen Verfahren unerwünschte Einsatz der billigeren Schwefelsäure würde dazu führen, dass bei der pH-Erniedrigung, so die Neutralisation in der MDA-Herstellung unter Einsatz von Natronlauge erfolgte, Natriumsulfat entstünde, das bei der anschließenden Elektrolyse im Anolytkreislauf angereichert würde. Da z.B. die Ionenaustauschermembranen gemäß Herstellerangaben nur bis zu einer bestimmten Natriumsulfat-Konzentration im Anolyten betrieben werden dürfen, müsste mehr Anolyt ausgeschleust werden als bei Einsatz von Salzsäure oder Chlorwasserstoff, dessen Reaktionsprodukt bei der pH-Erniedrigung das gewünschte Natriumchlorid ist.

In einer weiteren bevorzugten Ausführung der Erfindung beträgt die Alkalichloridkonzentration der Alkalichlorid-haltigen wässrigen Lösung, die aus Schritt A4) erhalten wird, 2 bis 24 Gew.-%, bevorzugt 5 bis 15 Gew.-%. Insbesondere wird die Alkalichlorid-haltige wässrige Lösung vor ihrem Einsatz in einer elektrochemischen Oxidation durch Wasserentzug und/oder durch Zugabe von Alkalichloriden in fester Form oder in Form konzentrierter Lösung auf einen Alkalichloridgehalt > 5 Gew.-%, bevorzugt > 10 Gew.-%, ganz besonders bevorzugt > 20 Gew.-% gebracht.

Das Verfahren der Alkalichlorid-Elektrolyse wird im Folgenden näher beschrieben. Die nachstehende Beschreibung ist in Bezug auf die Elektrolyse von Natriumchlorid beispielhaft anzusehen, da im erfindungsgemäßen integriertem MDI-Verfahren grundsätzlich jedes Alkalihydroxid bei der Neutralisation des Reaktionsgemischs der MDA-Herstellung zum Einsatz kommen kann, die Verwendung von Natronlauge bzw. der Anfall von Natriumchlorid in den vorgehenden Stufen des erfindungsgemäßen integrierten MDI-Verfahrens aber die bevorzugte Ausführung des Verfahrens ist.

Üblicherweise werden zur Elektrolyse von Natriumchlorid-haltigen Lösungen Membranelektrolyseverfahren eingesetzt (siehe hierzu Peter Schmittinger, CHLORINE, Wiley-VCH Verlag, 2000, Seiten 77-115). Hierbei wird eine zweigeteilte Elektrolysezelle eingesetzt, die aus einem Anodenraum mit einer Anode und einem Kathodenraum mit einer Kathode besteht. Anoden- und Kathodenraum werden von einer Ionenaustauschermembran getrennt.

In den Anodenraum wird eine Natriumchlorid-haltige Lösung mit einer Natriumchlorid-Konzentration von üblicherweise mehr als 300 g/l eingeführt. An der Anode wird das Chlorid-Ion zu Chlor oxidiert, das mit der abgereicherten Natriumchlorid-haltigen Lösung (ca. 200 g/l) aus der Zelle geführt wird. Die Natrium-Ionen wandern unter Einfluss des elektrischen Feldes durch die Ionenaustauschermembran in den Kathodenraum. Bei dieser Wanderung nimmt jedes mol Natrium bei den üblicherweise eingesetzten Membranen Wasser mit.

Der Kathodenkammer wird üblicherweise eine "verdünnte" Natronlauge zugeführt. An der Kathode wird Wasser unter der Bildung von Wasserstoff und Hydroxid-Ionen elektrochemisch reduziert. Durch diese Reaktion wird auf der Kathodenseite der Zelle Wasser verbraucht, dabei durch die im Kathodenraum verbleibenden Hydroxid-Ionen und die über die Ionenaustauschermembran in den Kathodenraum gelangenden Natrium-Ionen "zusätzliche" Natronlauge erzeugt. Alternativ kann als Kathode auch eine Gasdiffusionselektrode eingesetzt werden, an der Sauerstoff mit Elektronen zu Hydroxid-Ionen umgesetzt wird und kein Wasserstoff entsteht. Somit können beispielweise Gasdiffusionselektroden gemäß der DE 10 2005 023615 A1 eingesetzt werden. Der Betrieb der Gasdiffusionselektrode kann gemäß der EP 1 033 419 A1, US 6 117 286 B2 oder WO 2001/057290 A1 erfolgen.

Bevorzugt ist daher ein Verfahren, das dadurch gekennzeichnet ist, dass die elektrochemische Oxidation gemäß Schritt C) nach einer Alkali-Chlorid-Elektrolyse unter Einsatz von Ionenaustauschermembranen und/oder unter Einsatz von Gasdiffusionselektroden als Kathode erfolgt.

Üblicherweise ist es Ziel, möglichst hohe Konzentrationen an Natronlauge zu erreichen, da handelsübliche Natronlauge als 50 Gew.-%ige Lauge gelagert und transportiert wird. Handelsübliche Membranen sind jedoch derzeit nicht gegen eine Lauge mit einer höheren Konzentration als ca. 35 Gew.-% beständig, so dass die Natronlauge durch thermische Eindampfung aufkonzentriert werden muss. Bei dem Einsatz handelsüblicher Membranen wird üblicherweise ein Teilstrom der erzeugten "konzentrierten" Natronlauge ausgeschleust und der verbleibende Strom mit Wasser verdünnt und wieder auf die Kathodenseite der Natriumchlorid-Elektrolyse zurückgeführt, dabei die Natronlauge-Konzentration im Auslauf der Zelle auf 31-32 Gew.-% gehalten, dies bei einer Einlaufkonzentration der Lauge von ca. 30 % und einer Stromdichte von 4 kA/m².

Bei der skizzierten Natriumchlorid-Elektrolyse wird über die Natriumchlorid-haltige Lösung Wasser in den Anolyten eingebracht, jedoch nur Wasser über die Membran in den Katholyten ausgetragen. Wird mehr Wasser über die Natriumchlorid-haltige Lösung eingebracht als durch die Membran zum Katholyten transportiert werden kann, verarmt der Anolyt an Natriumchlorid und die Elektrolyse kann nicht kontinuierlich betrieben werden, denn bei sehr geringen Natriumchlorid-Konzentrationen würde die Nebenreaktion der Sauerstoffbildung einsetzen. Zur Vermeidung dieser Nebenreaktion wird die Elektrolyse bei Einsatz üblicher Membran-Elektrolysenzellen so gefahren, dass der aus der Zelle abgeführte Anolyt eine Konzentration von ca. 200 g/l Natriumchlorid aufweist, die Konzentration des abgeführten Anolyten dann durch Zufuhr von festem Natriumchlorid oder einer hochkonzentrierten Natrium-Chlorid-Lösung wieder auf die Sollstärke von ca. 300 g/l erhöht und in den Anodenraum zurückgeführt wird, es zweckmäßig sein kann, zur Vermeidung der Anreicherungen unerwünschter Bestandteile einen Teilstrom von 1-5% des abgereicherten Anolytstromes auszuschleusen.

Bei dem erfindungsgemäßen integrierten MDI-Verfahren wird in einer bevorzugten Ausführung ein besonders wirtschaftlicher Einsatz der bei der MDA-Herstellung anfallenden Natriumchlorid-haltigen Lösungen in der Elektrolyse dadurch erreicht, dass nach erfolgter Aufkonzentrierung der Natriumchlorid-haltigen Lösungen bei deren Elektrolyse der Wassertransport über die Membran erhöht wird. Dies erfolgt durch die Auswahl geeigneter Membranen, wie z.B. in der US 6 984 669 B2 beschrieben, wobei Ionenaustauschermembranen eingesetzt werden, die einen Wassertransport je mol Natrium von mehr als 3 mol H₂O/mol Natrium, bevorzugt > 4 mol H₂O/mol Natrium, besonders bevorzugt > 5 mol H₂O/mol Natrium aufweisen.

Bevorzugt ist folglich eine Ausführung des Verfahrens, **dadurch gekennzeichnet, dass** die Alkali-Chlorid-Elektrolyse nach dem Membranelektrolyseverfahren unter Einsatz einer Ionenaustauschermembran durchgeführt wird, wobei das Alkalichlorid der Alkalichlorid-haltigen Lösung Natriumchlorid ist und die Ionenaustauschermembran eine Wassertransportkapazität von > 3 mol Wasser / mol Natrium, bevorzugt > 4 mol Wasser / mol Natrium, besonders bevorzugt > 5 mol Wasser / mol Natrium aufweist.

Der Effekt eines erhöhten Wassertransportes besteht zum einen darin, dass auf die sonst übliche Wasserzugabe zur Aufrechterhaltung der Laugenkonzentration verzichtet werden kann, zum anderen darin, dass bei gleicher Ablaufkonzentration ein verbesserter Umsatz des eingesetzten Natriumchlorids erzielt wird. So werden z.B. bei Einsatz einer Membran mit einem Wassertransport von 6,0 mol H₂O/mol bei einer Einsatzkonzentration der Natriumchlorid-haltigen Lösung von 24 Gew.-% und einer Ablaufkonzentration von 16,3 Gew.-% eine Laugenkonzentration von 30,4 Gew.-% und in einem einmaligen Durchgang eine Recyclingquote von NaCl von 66 %, bei Kreislaufführung eine Recyclingquote von fast 100 % erreicht. Hierfür notwendig ist, dass das NaCl in einer wässrigen Lösung mit einer Konzentration von 24 vorliegt, und die abgereicherte NaCl-Lösung, die aus der Zelle kommt, zurückgeführt und vor ihrem Wiedereinsatz mit festem NaCl bis zu einer Konzentration von 24 Gew.-% konzentriert wird. Zur Vermeidung von Anreicherungen von Verunreinigungen wird ca. 1% der abgereicherten aus der Zelle gelangenden NaCl-Lösung ausgeschleust.

Eine weitere Möglichkeit, den Wassertransport zu beeinflussen, besteht darin, die Betriebsbedingungen der Elektrolyse der NaCl-Lösungen dahingehend zu modifizieren, dass die NaCl-Konzentration erniedrigt und / oder die Natronlauge-Konzentration erniedrigt wird. Ebenfalls denkbar ist, dass bei niedriger Solekonzentration und niedriger Natronlaugekonzentration ein erhöhter Wassertransport stattfindet. Eine weitere Möglichkeit ist, die Stromdichte der Elektrolyse zu erhöhen, um damit einen höheren Wassertransport zu ermöglichen, wobei dies auch bei veränderten Konzentrationen von Natronlauge und NaCl-Lösung erfolgen kann. Dies erfolgt dann bevorzugt mit dem Einsatz von Anoden, die eine größere innere Oberfläche aufweisen, so dass die lokale Stromdichte deutlich niedriger ist.

Bei der skizzierten bevorzugten Form des neuen MDI-Verfahrens unter der Verwendung von Ionenaustauschermembranen, die eine Wassertransportkapazität > 4,0 mol H₂O/ mol Natrium aufweisen, beträgt die Stromdichte auf die Membranfläche berechnet insbesondere 2 bis 6 kA/m². Besonders bevorzugt werden Anoden mit größerer Oberfläche eingesetzt. Unter Anoden mit größerer Oberfläche sind solche zu verstehen, bei denen die physikalische Oberfläche deutlich höher ist als die geometrische Oberfläche, d.h. berechnet nach den äußeren Abessungen der Elektrode. Anoden mit größerer Oberfläche sind z.B. Schaum- oder Filz-artig aufgebaute Elektroden. Hierdurch wird anodisch eine sehr große Elektrodenoberfläche angeboten und die lokale Stromdichte stark erniedrigt. Die Oberfläche der Anode ist bevorzugt so zu wählen, dass die lokale Stromdichte bezogen auf die physikalische Oberfläche der Elektrode kleiner als 3 kA/m² ist. Je größer die Oberfläche und je niedriger die lokale Stromdichte, desto gering kann die Natriumchlorid-Konzentration in der Sole gewählt werden und desto höher ist der Anteil an Natriumchlorid aus der Sole, der in einem Durchgang der Sole als Anolyt recycelt werden kann.

Die Alkalichlorid-Elektrolyse gemäß Schritt C) sollte besonders bevorzugt so betrieben werden, dass die Alkalichlorid-Konzentration der aus der Zelle gelangenden Alkalichlorid-Lösung 100 bis 280 g/l Natriumchlorid und/oder dass die Konzentration der Alkalilauge, die aus der Zelle gelangt 13 bis 33 Gew.-% beträgt. Ganz besonders bevorzugt sind Konzentrationen, die den Betrieb der Zelle bei niedrigerer Spannung ermöglichen. Hierzu sollte die Konzentration der aus der Zelle gelangenden Alkalichlorid-Lösung vorzugsweise zwischen 110 bis 220 g/l Alkalichlorid und/oder die Konzentration der Alkalilauge, die aus der Zelle gelangt, 20 bis 30 Gew.-% betragen.

Bei dem neuen integrierten MDI-Verfahren unter Einsatz einer Natriumchlorid-Elektrolyse wird die Elektrolyse bevorzugt bei einer Temperatur von 70 bis 100°C, vorzugsweise bei 80 bis 95°C und bei einem Absolutdruck von insbesondere 1 bis 1,4 bar, bevorzugt bei einem Absolutdruck von 1,1 bis 1,2 bar, betrieben, wobei die Druckverhältnisse zwischen Anoden- und Kathodenraum insbesondere so gewählt werden, dass der Druck im Kathodenraum höher ist als der Druck im Anodenraum. In einer besonders bevorzugten Ausführung beträgt der Differenzdruck zwischen Kathoden- und Anodenraum 20 bis 150 mbar, ganz besonders bevorzugt 30 bis 100 mbar.

Bei niedrigerer Alkalichlorid-Konzentration können auch spezielle Anodencoatings eingesetzt werden. Insbesondere kann das Coating der Anode neben Rutheniumoxid auch weitere Edelmetallkomponenten der 7. und 8. Nebengruppe des Periodensystems der Elemente enthalten. Beispielsweise kann das Anodencoating mit Palladiumverbindungen dotiert werden. Ebenso sind Coatings auf Basis von Diamanten einsetzbar.

Wird die Alkalichlorid-Elektrolyse in dem neuen MDI-Verfahren allein zur Aufbereitung der bei der MDA-Herstellung anfallenden Alkalichlorid-haltigen Lösungen eingesetzt, sind diese Lösungen vor ihrem Einsatz in der Elektrolyse durch Alkalichloridzugabe oder durch Wasserentzug zu konzentrieren, bevorzugt auf eine Alkalichlorid-Konzentration von > 10 Gew.-%. Bei Wasserentzug erfolgt die Konzentrierung der bei der MDA-Herstellung anfallenden Natriumchlorid-haltigen Lösungen bevorzugt mittels Membranverfahren, wie z.B. durch Umkehrosmose oder Membrandestillationsverfahren (siehe MELIN; RAUTENBACH, Membranverfahren; SPRINGER, BERLIN, 2003, Seiten 495-530). Durch Kombination von erfindungsgemäßem Betrieb der Elektrolysezellen und Aufkonzentrierungsverfahren können theoretisch bis zu 100 % des Natriumchlorids aus dem NaCl-haltigen Wässern aus der MDA-Herstellung wieder gewonnen werden.

Das bei der Alkalichlorid-Elektrolyse erzeugte Chlor wird in bekannter Weise aufbereitet (siehe Ullmann's Enzyklopädie der technischen Chemie, 6.Auflage, Band 8, Seite 254 - 265), dann zumindest teilweise der Phosgensynthese des erfindungsgemäßen integrierten MDI-Verfahrens zugeführt.

Für die Phosgenierung des nach dem neuen integrierten MDI-Verfahren erzeugten MDAs wird mehr Chlor benötigt, als durch die Elektrolyse des in die MDA-Herstellung eingesetzten, in Form einer Alkalichlorid-haltigen wässrigen Lösung zurück gewonnenen HCl-Katalysators anfällt. So können bei einem A/F-Verhältnis von 3 und einem Protonierungsgrad von 30% ca. 25 % des benötigten Chlors aus der Rückführung und elektrochemischen Oxidation des neutralisierten Katalysators gewonnen werden, bei gleichem A/F-Verhältnis und einem Protonierungsgrad von 20 % nur ca. 17 %. Es muss daher bei dem neuen Verfahren weiteres Chlor für die Phosgensynthese bereitgestellt werden.

Diese zusätzliche Chlorbereitstellung kann nach allen bekannten Verfahren der Technik erfolgen, z.B. durch Einspeisung extern bezogenen Chlors oder durch eine im Verbund mit der MDI-Herstellung betriebenen Chlorherstellung aus dem bei der MDI-Herstellung anfallenden Chlorwasserstoff durch Direktoxidation nach dem Deacon-Verfahren oder nach Überführung des Chlorwasserstoffs in eine Salzsäure und deren Elektrolyse nach dem Diaphragmaverfahren oder unter Elektrolyse mit Einsatz einer Gasdiffusionselektrode als Kathode. Geeignete Verfahren sind z.B. beschrieben in WO2004/014845 A1 ("Integriertes Verfahren zur Herstellung von Isocyanaten und katalytische Oxidation von Chlorwasserstoff nach dem Deacon-Verfahren"), WO2000/073538 A1 und WO2002/018675 A2 ("Elektrochemische Oxidation einer wässrigen Lösung von Chlorwasserstoff unter Verwendung einer Gasdiffusionselektrode als Kathode") und speziell in EP 1 743 882 A1 ("Integriertes Verfahren zur Herstellung von Isocyanaten aus Phosgen und wenigstens einem Amin und elektrochemische Oxidation des dabei anfallenden Chlorwasserstoffs zu Chlor, wobei das Chlor zur Herstellung des Phosgens rückgeführt wird"), doch auch andere elektrochemische Recycling-Verfahren sind geeignet.

Die Bereitstellung des zusätzlich benötigten Chlors kann aber auch durch eine entsprechend dimensionierte Alkalichlorid-Elektrolyse erfolgen, wobei der bei der MDI-Synthese anfallende, nicht als Katalysator in der MDA-Herstellung des integrierten neuen MDI-Verfahrens benötigte Chlorwasserstoff dann als Einsatzstoff für einen EDC-Prozess Verwendung finden oder nach dem Stand der Technik über ein elektrochemisches Recycling-Verfahren zu Chlor aufbereitet werden oder, in Wasser absorbiert, als Salzsäure einer Verwendung zugeführt werden kann. Eine Übersicht über geeignete elektrochemische Recycling-Verfahren wird in dem Artikel "Chlorine Regeneration from Anhydrous Hydrogen Chloride" von Dennie Turin Mah, veröffentlicht in "12th International Forum Electrolysis in Chemical Chemistry - Clean and Efficient Processing Electrochemical Technology for Syntheses, Separation, Recycle and Environmental Improvement, October 11-15, 1998. Sheraton Sand Key, Clearwater Beach, FL", gegeben.

Bevorzugt erfolgt die Bereitstellung des zusätzlich benötigten Chlors in einer Alkalichlorid-Elektrolyse, deren Dimensionierung den möglichst vollständigen Einsatz der in Schritt A der MDA-Herstellung des erfindungsgemäßen integrierten MDI-Verfahrens anfallenden und anschließend durch Extraktion, Wasserdampfstrippung und Behandlung an Aktivkohle weitgehend von seinen organischen Verunreinigungen befreiten wässrigen Alkalichlorid-Lösungen zur Bereitung der Solelösungen gestattet, wobei in einer ganz besonders bevorzugten Ausführung der Bedarf an Verdünnungswasser für die auf der Kathodenseite der Natriumchlorid-Elektrolyse entstehenden Natronlauge zum einen durch das mit den Natriumionen auf die Kathodenseite gelangende Wasser gedeckt wird und zum anderen die Wassermenge des bei der MDA-Herstellung des erfindungsgemäßen integrierten MDI-Verfahrens anfallenden Abwasserstroms übersteigt. Dieser vollständige Einsatz wird speziell bei niedrigem ( < 30 % ) Protonierungsgrad in der MDA-Herstellung des neuen integrierten MDI-Verfahrens erreicht, bevorzugt ist ein Protonierungsgrad < 20 %, besonders bevorzugt < 15 %, ganz besonders bevorzugt < 10%. Die Kombination des Einsatzes spezieller Ionenaustauschermembranen, die gegenüber dem üblichen Stand der Technik hohe Wassertransporte über die Membranen zulassen, und den erfindungsgemäß niedrigen Protonierungsgraden bei der MDA-Herstellung führt mithin zu einer besonders bevorzugten Form des neuen integrierten MDI-Verfahrens. Diese besonders bevorzugte Form ist **dadurch gekennzeichnet, dass** - sieht man von Purgemengen ab - ein vollständiges Recycling des in der MDA-Herstellung eingesetzten sauren Katalysators erreicht wird, gleichzeitig der bei der MDA-Herstellung anfallende Abwasserstrom als Natriumchlorid-haltiges Abwasser vollständig in der NaCl-Elektrolyse eingesetzt und dort vollständig als Verdünnungswasser in der Natronlaugeherstellung genutzt werden kann, mithin in dem neuen integrierten MDI-Verfahren kein bei der MDA-Herstellung anfallendes Abwasser zur Aufbereitung außerhalb des integrierten MDI-Verfahrens abgegeben werden muss.

In einer weiteren bevorzugten Ausführung erfolgt die Bereitstellung des zusätzlich benötigten Chlors in einer Alkalichlorid- und einer HCl-Elektrolyse, wobei die Dimensionierung der Alkalichlorid-Elektrolyse den vollständigen Einsatz der in Schritt A der MDA-Herstellung des erfindungsgemäßen integrierten MDI-Verfahrens anfallenden und anschließend durch Extraktion, Wasserdampfstrippung und Behandlung an Aktivkohle weitgehend von seinen organischen Verunreinigungen befreiten wässrigen Alkali-chlorid-Lösungen zur Bereitung der Solelösungen gestattet, das darüber hinaus benötigte Chlor über eine HCl-Elektrolyse nach dem Diaphragmaverfahren und/oder unter Einsatz einer Gasdiffusionselektrode als Kathode bereit gestellt wird, und das bei der Alkalichlorid-Elektrolyse sowie der HCl-Elektrolyse entstehende Chlor in einer gemeinsamen Aufbereitung für den Einsatz in die Phosgenerzeugung des integrieren erfindungsgemäßen MDI-Verfahrens aufbereitet werden.

Das so im benötigten Umfang bereitgestellte Chlor wird mit Kohlenmonoxid zu Phosgen umgesetzt, wobei die nach dem Stand der Technik bekannten Verfahren, wie sie z.B. in Ullmanns Enzyklopädie der industriellen Chemie, 3.Auflage, Band 13, Seiten 494-500 dargestellt sind, zum Einsatz kommen können. Die im technischen Maßstab übliche Umsetzung von Kohlenmonoxid und Chlor an Aktivkohle als Katalysator bei Temperaturen von 250°C bis maximal 600°C wird bevorzugt, wobei besonders bevorzugt Rohrbündelreaktoren zum Einsatz kommen. Die Abführung der Reaktionswärmen der stark exothermen Reaktion kann auf unterschiedliche Weise erfolgen, beispielsweise durch ein flüssiges Wärmetauschmittel, wie z.B. in WO03/072237 A1 beschrieben, oder durch Siedekühlung über einen Sekundärkreislauf unter gleichzeitiger Nutzung der Reaktionswärmen zur Dampferzeugung, wie z.B. in US 4 764 308 A offenbart.

Die Umsetzung von Phosgen mit Polyaminen der Diphenylmethanreihe wird im großtechnischen Maßstab durchgeführt und ist vielfach beschrieben (s. z.B. Kunststoffhandbuch, Band 7 (Polyurethane), 3. neubearbeitete Auflage, Carl Hanser Verlag, München-Wien, S.76 ff (1993)). Für das erfindungsgemäße integrierte MDI-Verfahren sind zur Umsetzung der in Schritt A) erzeugten Polyamine der Diphenylmethanreihe alle nach dem Stand der Technik bekannten Phosgenierverfahren in flüssiger Phase geeignet, bevorzugt erfolgt die Umsetzung der in Schritt A) des neuen Verfahrens erzeugten Polyamine der Diphenylmethanreihe nach den in WO 2004/056756 A1 und EP 1 873 142 A1 offenbarten Verfahren, auf die hier ausdrücklich Bezug genommen wird. Besonders bevorzugt erfolgt die Umsetzung der in Schritt A) erzeugten Polyamine der Diphenylmethanreihe nach dem in EP 1 873 142 A1 offenbarten Verfahren.

Das in EP 1 873 142 A1 offenbarte Verfahren beinhaltet die Umsetzung der Polyamine der Diphenylmethanreihe mit Phosgen in mindestens drei Stufen, wobei die erste Stufe in einem dynamischen Mischer, die zweite Stufe in mindestens einem Reaktor und die dritte Stufe in mindestens einem Stofftrennapparat durchgeführt wird, und wobei der Druck in der zweiten Stufe größer oder gleich dem Druck in dem dynamischen Mischer ist und der Druck in dem mindestens einen Stofftrennapparat kleiner als der Druck in dem Reaktor der zweiten Stufe ist.

Nach der Lehre von EP 1 873 142 A1 erfolgt in der ersten Phosgenierstufe des offenbarten Verfahrens im wesentlichen die Umsetzung der primären Amingruppen der eingesetzten Polyamine der Diphenyl-methanreihe zu Carbamoylchloriden und Aminhydrochloriden, in der zweiten Stufe im wesentlichen die Umsetzung der in der ersten Stufe gebildeten Aminhydrochloride zu Carbamoylchloriden und in der dritten Stufe im wesentlichen die Spaltung der Carbamoylchloride zu Isocyanatgruppen und Chlorwasserstoff. Bei dem in EP 1 873 142 A1 offenbarten Verfahren wird die Umsetzung des Amins mit dem Phosgen in einem inerten Lösungsmittel, vorzugsweise Toluol oder Chlorbenzol, Dichlorbenzol oder Mischungen davon, und mit einem Phosgenüberschuss durchgeführt.

Gemäß der Lehre von EP 1 873 142 A1 erfolgt die erste Phosgenierstufe in einem dynamischen Mischer, bevorzugt in Mischer-Reaktoren, die beispielsweise in EP 0 291 819 B1 in Spalte 1 / Zeile 44 bis Spalte 2 / Zeile 49 in ihrer Funktion und in Verbindung mit den Figuren 1 und 2 in Spalte 4 7 Zeile 34 bis Spalte 5 / Zeile 46 in ihrem detaillierten Aufbau beschrieben sind, besonders bevorzugt in Misch-Reaktoren, wie sie in EP 0 830 894 B1 in den Absätzen 0005 bis 0011 / 0012 bis 0014 in ihrer Funktion und in Verbindung mit den Figuren 1 bis 4 in den Absätzen 0016 bis 0022 in ihrem detaillierten Aufbau beschrieben sind. Geeignet für den Einsatz in der ersten Phosgenierstufe sind aber grundsätzlich alle dynamischen Mischer, die durch mechanisch bewegte Teile eine intensive Durchmischung sicherstellen, beispielsweise Rotationsmischeinrichtungen und besonders mehrstufige Kreiselpumpen.

Bei dem Einsatz im neuen integrierten MDI-Verfahren beträgt insbesondere der Druck in dem dynamischen Mischer der ersten Phosgenierstufe bevorzugt 3 bis 70 bar, besonders bevorzugt 3 bis 35 bar. Die Temperatur in der ersten Stufe beträgt bevorzugt 80 bis 220 °C, besonders bevorzugt 90 bis 180 °C, wobei die Umsetzung in der ersten Stufe bevorzugt adiabat durchgeführt wird.

Analog zu dem in EP 1 873 142 offenbarten Verfahren wird bei dem neuen integrierten MDI-Verfahren die zweite Phosgenierstufe in mindestens einem Reaktor, d.h. einem für die Durchführung von chemischen Reaktionen geeigneten Verweilzeitapparat durchgeführt, der hydraulisch mit dem dynamischen Mischer der ersten Stufe verbunden betrieben wird. Der Druck in der zweiten Phosgenierstufe beträgt bevorzugt 3 bis 70, besonders bevorzugt 3 bis 37 bar. Die Temperatur in der zweiten Stufe beträgt bevorzugt 80 bis 220 °C, besonders bevorzugt 90 bis 180 °C. Als Reaktortypen für die zweite Stufe kommen Rohrreaktoren, Rührkessel oder auch nicht durchmischte Verweilzeitapparate in Betracht. Der Reaktor kann auch mit einem Umpumpkreislauf versehen sein, wobei dieser wiederum einen Wärmeaustauscher zur Einstellung der Reaktortemperatur enthalten kann. Besonders bevorzugt ist der Einsatz von Rohrreaktoren.

Gemäß der Lehre von EP 1 873 142 A1 erfolgt in der dritten Phosgenierstufe des in EP 1 873 142 A1 offenbarten Verfahrens zum einen die Umsetzung der Carbamoylgruppen zu den Isocyanatgruppen, zum anderen wird das Reaktionsgemisch in eine Gasphase und eine Flüssigphase getrennt. Die Gasphase enthält dabei im wesentlichen Chlorwasserstoff und in Abhängigkeit von Druck und Temperatur ggf. anteilig das im Überschuss eingesetzte Phosgen und Lösungsmittelbrüden. Analog zu dem in EP 1 873 142 A1 offenbarten Verfahren wird die dritte Phosgenierstufe bei dem neuen integrierten MDI-Verfahren bevorzugt bei einem Druck von 0,5 bis 20 bar, besonders bevorzugt von 0,5 bis 16 bar, betrieben, wobei hinter dem Reaktor der zweiten Phosgenierstufe über eine Armatur oder eine andere dafür geeignete Einrichtung auf den Druck des Stofftrennapparats entspannt, die Temperatur der dritten Phosgenierstufe auf 80 bis 220 °C eingestellt wird.

Als Reaktor für den Stofftrennapparat der dritten Phosgenierstufe, sind für das neue integrierte MDI-Verfahren neben Wärmeaustauschern mit separatem Gasaustrag Rührkessel, Rührkesselkaskaden, Lochbodenkolonnen oder andere Apparate zur Stofftrennung geeignet, besonders geeignet ist ein (Reaktions-)Turm gemäß DE 37 36 988 C1 / Spalte 3, Zeilen 2 -64. Der Stofftrennapparat der dritten Phosgenierstufe kann auch benutzt werden, um das überschüssige Phosgen aus der Reaktionsmischung zu entfernen. Der Stofftrennapparat der dritten Stufe kann ebenfalls nachteilig groß sein. In diesem Fall kann der Stofftrennapparat der dritten Stufe auch durch zwei oder mehrere gleichartige oder unterschiedliche Apparate, bevorzugt eine Kombination von Wärmeaustauscher mit separatem Gasaustrag und (Reaktions-)Turm bzw. (Reaktions-)Turm /Türmen und (Reaktions-)Kolonne, die bevorzugt in Serie geschaltet sind, realisiert werden.

Das aus der Phosgenierung ausgetragene Reaktionsgemisch wird anschließend nach den üblichen Methoden zur Entfernung gegebenenfalls noch vorhandenen Phosgens und zur Abtrennung des Lösungsmittels aufgearbeitet. Weitere Reinigungsschritte können sich anschließen.

Aus den in der Phosgenierung sowie den in der nach geschalteten Entphosgenierung erzeugten Brüden werden dann in an sich bekannter Weise Phosgen, Chlorwasserstoff und Lösungsmittel abgetrennt und ggf. an geeignete Stellen des Verfahrens zurückgeführt. Bevorzugt werden die Brüden zu ihrer Aufbereitung vereinigt und erst dann in ihre Komponenten aufgetrennt, wobei bevorzugt das in DE 102 60 084 A1 offenbarte Verfahren angewandt wird, auf die zitierte Anmeldung ausdrücklich Bezug genommen wird.

Wie oben dargestellt kann der in dem neuen integrierten MDI-Verfahren anfallende Chlorwasserstoff, ggf. nach einer zusätzlich durchgeführten Nachreinigung, in eine Chlorherstellung nach dem Deacon-Verfahren oder nach seiner Überführung in eine Salzsäure durch deren Elektrolyse überführt werden, ist sein Einsatz in einem EDC-Prozess oder seine Abgabe in Form einer Salzsäure ebenfalls möglich.

In einer bevorzugten Ausführung des neuen integrierten MDI-Verfahrens wird ein Teilstrom des in der Phosgenierung erzeugten Chlorwasserstoffs, bevorzugt in Form einer 25-30 Gew.-%igen Salzsäure, als Katalysator in der MDA-Herstellung des integrierten Verfahrens eingesetzt, und wird der verbleibende Chlorwasserstoff nachgereinigt und wie oben dargelegt einem elektrochemischen Recycling-Verfahren zur Chlorherstellung zugeführt. Die Nachreinigung des Chlorwasserstoffs erfolgt dabei bevorzugt nach einem der in WO 2003/089 370 A1 und WO 2006/089 877 A1 offenbarten Verfahren, wobei in einer besonders bevorzugten Form beide Verfahren in serieller Anordnung zur Anwendung kommen.

Die in den vorstehenden Kapiteln beispielhaft aufgeführten Ausführungsformen der Stufen des erfindungsgemäßen integrierten MDI-Verfahrens können im Einzelnen in anderer, dem Fachmann grundsätzlich bekannter Weise durchgeführt werden, ohne dass die erfindungsgemäße Neuerung hiervon berührt wird.

Die nachfolgenden Beispiele sollen das erfindungsgemäße integrierte MDI-Verfahren illustrieren ohne es jedoch einzuschränken.

### Beispiele

### Beispiel 1

12,22 t/h einer Mischung, enthaltend anteilmäßig Abwässer und Kondensate aus den Stufen einer MDA-Herstellung, die folgende Schritte aufweist, dass nämlich
a) Anilin und Formaldehyd in einem A/F-Verhältnis von 3 in Gegenwart von 30 Gew.-%iger Salzsäure als Katalysator bei einem Protonierungsgrad von 10% umgesetzt werden,
b) das Reaktionsgemisch mit Natronlauge neutralisiert wird,
c) die organische Phase abgetrennt wird,
d) die organische Phase mit heißem Wasser gewaschen wird und
e) aus der organischen Phase überschüssiges Anilin destillativ entfernt, die Kondensate ohne weitere Aufarbeitung dem Sammelabwasser ( alle Abwässer der Schritte a)-d)) zugeführt werden,
mit einem Gehalt an 0.24 Gew.-% MDA, 33.8 Gew.-% Anilin und 4.9 Gew.-% Natriumchlorid werden über einen Wärmeaustauscher auf Temperaturen > 80°C aufgeheizt und zu ihrer Aufbereitung einer Separationsstufe (Schritt g)) und nachfolgend einer zweistufigen, im Gegenstrom mit MDA-freiem Anilin als Extraktionsmittel betriebenen Extraktionsstufe zugeführt. Hierbei wird
- das mit 1,5 t/h zugeführte MDA-freie Anilin vor seiner Einspeisung in die Extraktionsstufe auf eine Temperatur > 50 °C aufgeheizt,
- das frisch eingesetzte wie das die erste und zweite Stufe der Extraktionsstufe verlassende Anilin intensiv unter einem Energieeintrag von 0,1 kW/m³ Abwasser mit dem im Gegenstrom geführten Abwasser bzw. der eingesetzten Mischung vermischt, dann in den Mixern nach geschalteten Settlern mit einer mittleren Verweilzeit von 20 Minuten unter Einhaltung einer Temperatur von > 80 °C wieder abgetrennt.

Nach der Extraktion das Abwasser enthält das Abwasser < 1 ppm MDA, ist ebenfalls frei von Aminalen und Aminobenzylaminen. Sein Anilingehalt von 3,1 Gew.-% entspricht dem Lösungsgleichgewicht.

Zur weiteren Aufbereitung wird das extrahierte Abwasser mit 30 Gew.-%iger Salzsäure auf einen pH-Wert von 7,2 gestellt, dann einer destillativen Anilinabtrennung zugeführt, dort bei 450 mbar und 80°C von anhaftendem Anilin und beinhaltenden Leichtsiedern befreit, wobei die bei der destillativen Anilinabtrennung anfallenden Kondensate einer weiteren Leichtsiederabtrennung zugeführt, so aufbereitet, als Verdünnungswasser in den Schritt b) und/oder als Waschwasser auf den Schritt d) der MDA-Herstellung zurückgeführt werden.

Das die destillative Anilinabtrennung mit 5,6 t/h verlassende Abwasser mit einem NaCl-Gehalt von 12,2 Gew.-% wird über einen Wärmeaustauscher auf 40°C temperiert, über ein A-Kohle-Kontrollbett geführt, dann der Solebereitung einer NaCl-Elektrolyse zugeführt, wobei die in die Solebereitung eingespeiste Lösung einen TOC-Wert von 19 ppm aufweist.

Die Elektrolyse wird in 302 Zellen mit einer Anodenfläche von je 2,71m² durchgeführt, wobei die Stromdichte 4 kA/m², die Zellspannung 2,0 V, die Temperatur im Auslauf Kathodenseite 88 °C, die Temperatur im Auslauf Anodenseite 89 °C beträgt. Die Elektrolysezellen sind mit Standard Anodencoating der F. Denora, Deutschland ausgerüstet, als Ionenaustauschermembranen finden Membranen des Typs N2010 der Fa. DuPont Verwendung, und durch die Anodenkammern der Zellen werden 200 kg/h, durch die Kathodenkammern 75 kg/h je Zelle umgepumpt. Als Kathode wird eine Gasdiffusionselektrode gemäß DE 10 2005 023615 A1 eingesetzt.

Die Konzentration der den Anodenkammern zugeführten NaCl-Lösung beträgt 22 Gew.-% NaCl. Aus den Anodenkammern wird eine 15,1 %ige Lösung entnommen. Von diesem Strom werden 0,406 t/h verworfen. Der den Anodenkammern entnommenen NaCl-Lösung werden 5,6 t/h an MDA-Abwassser mit einem NaCl-Gehalt von 12,2 %, 6,51 t/h festes Natriumchlorid sowie 8,07 t/h weiteres Wasser zugegeben. Der Wassertransport über die eingesetzten Membranen beträgt 5,3 mol Wasser je mol Natrium.

Die Konzentration der in die Kathodenseiten eingespeisten Natronlauge beträgt 30 Gew.-%, die der den Kathodenseiten entnommenen Natronlauge beträgt 32,0 Gew.-%. 15,26 t/h der 32,0 Gew.-%igen Lauge werden dem Ablaufstrom der Kathodenseite entnommen, der Rest mit 1,43 t/h Wasser aufgestockt und wieder den Kathodenseiten der Elektrolysezellen zugeführt. 1,458 t/h an 32 Gew.-%iger NaOH werden in die MDA-Herstellung zurückgeführt, die verbleibenden Menge an Natronlauge steht zur Verwendung in anderen Prozessen zur Verfügung.

Auf Grund der hohen Wassertransportkapazität der eingesetzten Membranen ist nur eine sehr geringe zusätzliche Verdünnung der erzeugten Natronlauge erforderlich, wird die Verdünnung vorwiegend über den Wassertransport der Membranen erreicht, werden 11,65 t/h Wasser über die Membranen in die Kathodenseiten eingetragen, wird das gesamte Wasser aus dem MDA-Abwasser in die Elektrolyse zur Verdünnung der bei der Elektrolyse erzeugten Natronlauge genutzt.
4,32 t/h Chlor werden mit CO an A-Kohle bei einem CO-Überschuss von 7 mol-% zu Phosgen umgesetzt und in eine 45 Gew.-%ige Lösung von Phosgen in Monochlorbenzol überführt.
20 t/h einer 30 Gew.-%igen Lösung eines gemäß den Schritten a) - e) hergestellten MDA einer mittleren Molmasse von 224 g/mol in Monochlorbenzol mit einer Temperatur von 80°C und 26,7 t/h einer 45 Gew.-%igen Lösung von Phosgen in Monochlorbenzol mit einer Temperatur von - 5°C °C werden in einem Mischer-Reaktor gemäß EP-A-0 830 894 kontinuierlich unter einem Leistungseintrag von 52 kW vermischt und zur Reaktion gebracht. Der Druck in dem Misch-Reaktor beträgt 13,5 bar.

Nach einer mittleren Verweilzeit von 2,1 sec im Mischerreaktor wird das Reaktionsgemisch über das Laufrad des Mischerreaktors in einen anschließenden, bei 14 bar betriebenen, über einen Heizmantel beheizbaren Rohrreaktor gefördert, in diesem mit einer Verweilzeit von 120 sec bei > 120 °C gehalten, dann das Reaktionsgemisch über ein Regelventil in einen bei einem Kopfdruck von 1,7 bar betriebenen, beheizbaren, über Lochböden gekammerten Reaktionsturm als Phasentrennapparat entspannt.

Das Reaktionsgemisch wird in den Fuß des Reaktionsturms gespeist und auf seinem Weg durch den Apparat über Segmentbeheizungen gleichmäßig aufgeheizt, so dass die separat abgeführte Gasphase wie auch die Flüssigphase den Apparat mit einer Temperatur von 115°C verlassen.

Die abgezogene Gasphase (18,65 t/h ) enthält ein Gemisch aus Phosgen ( 5,64 t/h, 30,2 Gew.%), Chlorwasserstoff ( 4,37 t/h, 21,6 Gew.%), Monochlorbenzol ( 8,97 t/h, 48,10 Gew.% ) sowie geringe Mengen verschiedener Leichtsieder (Tetrachorkohlenstoff, Chloroform, Stickstoff, Kohlenmonoxid, Kohlendioxid) und wird einer Chlorwasserstoff/Phosgen-Trennung nach bekannter Art zugeführt. 0,426 t/h des abgetrennten und gereinigten Chlorwasserstoff werden in 0,99 t/h Wasser aufgenommen und in Form der so bereiteten 30 Gew.-%igen Salzsäure der MDA-Herstellung als Katalysator zugeführt.

Die aus dem Turm überlaufende flüssige Phase ( 28,05 t/h) enthält MDI ( 7,55 t/h, 26,9 Gew-%), Monochlorbenzol, Phosgen und geringe Mengen gelösten Chlorwasserstoffs, die flüssige Phase wird entsprechend dem Stand der Technik vom Chlorwasserstoff, Phosgen und Monochlorbenzol befreit und thermisch nachbehandelt. Das so hergestellte Gemisch aus Di- und Polyisocyanaten der Diphenylmethanreihe ist durch folgende Produkteigenschaften gekennzeichnet:
- Viskosität / 25°C: 49 mPa*s
- NCO-Gehalt 31,7 %
- Farbe E 430 ¹⁾ 0,134
- Farbe E 520 ¹⁾ 0,026
¹⁾ Von dem erhaltenen Isocyanat wurden 1,0 g in Chlorbenzol aufgelöst und mit Chlorbenzol zu 50 ml verdünnt. Die Extinktion der so erhaltenen Lösung wurde mit einem Photometer / Dr. Lange LICO 300 bei den beiden Wellenlängen 430 nm und 520 nm bestimmt.

### Beispiel 2:

8,3 t/h einer Mischung, enthaltend anteilmäßig Abwässer und Kondensate aus den Stufen einer MDA-Herstellung, bei der
f) Anilin und Formalin in einem A/F-Verhältnis von 2,1 in Gegenwart von 30 Gew.-%iger Salzsäure als Katalysator bei einem Protonierungsgrad von 15% umgesetzt werden,
g) das Reaktionsgemisch mit Natronlauge neutralisiert wird,
h) die organische Phase abgetrennt wird,
i) die organische Phase mit heißem Wasser gewaschen wird und
j) aus der organischen Phase überschüssiges Anilin destillativ entfernt, die Kondensate ohne weitere Aufarbeitung dem Sammelabwasser ( alle Abwässer der Schritte f) - i)) zugeführt werden,
mit einem Gehalt an 0.14 Gew.-% MDA, 16.2 Gew.-% Anilin und 6.5 Gew.-% Natriumchlorid werden über einen Wärmeaustauscher auf Temperaturen > 80°C aufgeheizt und zu ihrer Aufbereitung einer Separationsstufe (Schritt h)) und nachfolgend einer zweistufigen, im Gegenstrom mit MDA-freiem Anilin als Extraktionsmittel betriebenen Extraktionsstufe zugeführt. Hierbei wird
- das mit 1,0 t/h zugeführte MDA-freie Anilin vor seiner Einspeisung in die Extraktionsstufe auf Temperaturen > 75 °C aufgeheizt,
- das frisch eingesetzte wie das die erste und zweite Stufe der Extraktionsstufe verlassende Anilin intensiv unter einem Energieeintrag von > 0,1 kW/m³ Abwasser mit dem im Gegenstrom geführten Abwasser bzw. der eingesetzten Mischung vermischt, dann in den Mixern nach geschalteten Settlern mit einer mittleren Verweilzeit von 20 Minuten unter Einhaltung einer Temperatur von > 80 °C wieder abgetrennt.

Nach der Extraktion das Abwasser enthält das Abwasser < 11,5 ppm MDA, ist ebenfalls weitgehend frei von Aminalen und Aminobenzylaminen. Sein Anilingehalt von 3,1 Gew.-% entspricht dem Lösungsgleichgewicht.

Zur weiteren Aufbereitung wird das extrahierte Abwasser mit 30 Gew.-%iger Salzsäure auf einen pH-Wert von 7,2 gestellt, dann einer destillativen Anilinabtrennung zugeführt, dort bei 450 mbar und 80°C von anhaftendem Anilin und beinhaltenden Leichtsiedern befreit, wobei die bei der destillativen Anilinabtrennung anfallenden Kondensate einer weiteren Leichtsiederabtrennung zugeführt, so aufbereitet, als Verdünnungswasser in den Schritt g) und/oder als Waschwasser auf den Schritt i) der MDA-Herstellung zurückgeführt werden.

Das die destillative Anilinabtrennung mit 4,98 t/h verlassende Abwasser mit einem NaCl-Gehalt von 12,56 Gew.-% wird über einen Wärmeaustauscher auf 40°C temperiert, über ein A-Kohle-Kontrollbett geführt, dann der Solebereitung einer NaCl-Elektrolyse zugeführt, wobei die in die Solebereitung eingespeiste Lösung einen TOC-Wert von 24 ppm aufweist.

Die Elektrolyse wird in 115 Zellen mit einer Anodenfläche von je 2,71m² durchgeführt, wobei die Zellspannung 2,0 V, die Temperatur im Auslauf Kathodenseite 88 °C, die Temperatur im Auslauf Anodenseite 89 °C beträgt. Die Elektrolysezellen sind mit Standard Anodencoating der F. Denora, Deutschland ausgerüstet, als Ionenaustauschermembranen finden Membranen des Typs N2010 der Fa. DuPont Verwendung, und durch die Anodenkammern der Zellen werden 200 kg/h, durch die Kathodenkammern 75 kg/h je Zelle umgepumpt. Als Kathode wird eine Gasdiffusionselektrode gemäß DE 10 2005 023615 A1 eingesetzt.

Die Konzentration der den Anodenkammern zugeführten NaCl-Lösung beträgt 22 Gew.-% NaCl. Aus den Anodenkammern wird eine 15,1 %ige Lösung entnommen, davon werden 0,155 t/h als Purge entnommen, anschließend der den Anodenkammern entnommenen NaCl-Lösung 4,98 t/h an MDA-Abwassser mit einem NaCl-Gehalt von 12.56 %, 2,115 t/h festes Natriumchlorid sowie 0,51 t/h weiteres Wasser zugegeben. Der Wassertransport über die eingesetzten Membranen beträgt 5,2 mol Wasser je mol Natrium.

Die Konzentration der in die Kathodenseiten eingespeisten Natronlauge beträgt 30 Gew.-%, die der den Kathodenseiten entnommenen Natronlauge beträgt 32,0 Gew.-%. 5,775 t/h der 32,0 Gew.-%igen Lauge werden dem Ablaufstrom der Kathodenseite entnommen, der Rest mit 0,594 t/h Wasser aufgestockt und wieder den Kathodenseiten der Elektrolysezellen zugeführt. Auf Grund der hohen Wassertransportkapazität der eingesetzten Membranen ist nur eine sehr geringe zusätzliche Verdünnung der erzeugten Natronlauge erforderlich, wird die Verdünnung vorwiegend über den Wassertransport der Membranen erreicht, werden 4,355 t/h Wasser über die Membranen in die Kathodenseiten eingetragen, wird mithin alles des mit dem MDA-Abwasser in die Elektrolyse gelangenden Wassers zur Verdünnung der bei der Elektrolyse erzeugten Natronlauge genutzt.

1,649 t/h des in der NaCl-Elektrolyse erzeugten Chlors werden mit 1,963 t/h in einer konventionellen HCl-Elektrolyse nach dem Stand der Technik erzeugten Chlors versetzt und gemeinsam mit CO an A-Kohle bei einem CO-Überschuss von 7 mol-% zu Phosgen umgesetzt und in eine 45 Gew.-%ige Lösung von Phosgen in Monochlorbenzol überführt.

16,67 t/h einer 30 Gew.-%igen Lösung eines gemäß den Schritten f) - j) hergestellten MDA einer mittleren Molmasse von 232 g/mol in Monochlorbenzol mit einer Temperatur von 80°C und 22,22 t/h einer 45 Gew.-%igen Lösung von Phosgen in Monochlorbenzol mit einer Temperatur von - 5°C °C werden in einem Mischer-Reaktor gemäß EP-A-0 830 894 kontinuierlich unter einem Leistungseintrag von 58 kW vermischt und zur Reaktion gebracht. Der Druck in dem Misch-Reaktor beträgt 13,8 bar.

Nach einer mittleren Verweilzeit von 2,5 sec im Mischerreaktor wird das Reaktionsgemisch über das Laufrad des Mischerreaktors in einen anschließenden, bei 14,2 bar betriebenen, über einen Heizmantel beheizbaren Rohrreaktor gefördert, in diesem mit einer Verweilzeit von 120 sec bei > 120 °C gehalten, dann das Reaktionsgemisch über ein Regelventil in einen bei einem Kopfdruck von 1,7 bar betriebenen, beheizbaren, über Lochböden gekammerten Reaktionsturm als Phasentrennapparat entspannt.

Das Reaktionsgemisch wird in den Fuß des Reaktionsturms gespeist und auf seinem Weg durch den Apparat über Segmentbeheizungen gleichmäßig aufgeheizt, so dass die separat abgeführte Gasphase wie auch die Flüssigphase den Apparat mit einer Temperatur von 114°C verlassen.

Die abgezogene Gasphase (15,70 t/h ) enthält ein Gemisch aus Phosgen ( 4,68 t/h, 29,82 Gew.%), Chlorwasserstoff ( 3,60 t/h, 22,91 Gew.%), Monochlorbenzol ( 7,42 t/h, 47,27 Gew.% ) sowie geringe Mengen verschiedener Leichtsieder (Tetrachorkohlenstoff, Chloroform, Stickstoff, Kohlenmonoxid, Kohlendioxid) und wird einer Chlorwasserstoff/Phosgen-Trennung nach bekannter Art zugeführt. 0,39 t/h des abgetrennten und gereinigten Chlorwasserstoff werden in 0,91 t/h Wasser aufgenommen und in Form der so bereiteten 30 Gew.-%igen Salzsäure der MDA-Herstellung als Katalysator zugeführt.

Die aus dem Turm überlaufende flüssige Phase ( 23,19 t/h) enthält MDI ( 6,25 t/h, 26,95 Gew-%), Monochlorbenzol, Phosgen und geringe Mengen gelösten Chlorwasserstoffs, die flüssige Phase wird entsprechend dem Stand der Technik vom Chlorwasserstoff, Phosgen und Monochlorbenzol befreit und thermisch nachbehandelt. Das so hergestellte Gemisch aus Di- und Polyisocyanaten der Diphenylmethanreihe ist durch folgende Produkteigenschaften gekennzeichnet:

| | |
|---|---|
| Viskosität / 25°C: | 132 mPa*s |
| NCO-Gehalt: | 31,2 % |
| Farbe E 430 ¹⁾ | 0,182 |
| Farbe E 520 ¹⁾ | 0,035 |

| | |
|---|---|
| ¹⁾ Von dem erhaltenen Isocyanat wurden 1,0 g in Chlorbenzol aufgelöst und mit Chlorbenzol zu 50 ml verdünnt. Die Extinktion der so erhaltenen Lösung wurde mit einem Photometer / Dr. Lange LICO 300 bei den beiden Wellenlängen 430 nm und 520 nm bestimmt. | |

## Patentansprüche

1. Verfahren zur Herstellung von Methylen-diphenyl-diisocyanaten, wenigstens umfassend die Schritte
A1) Umsetzung von Anilin mit Formaldehyd in Gegenwart von Salzsäure zu einem Reaktionsgemisch enthaltend Di- und Polyamine der Diphenylmethanreihe ("MDA"), gegebenenfalls zumindest teilweise unter Entfernung des mit den Edukten eingetragenen oder bei der Umsetzung entstehenden Wassers aus dem Reaktionsgemisch,
A2) Neutralisation des gemäß Schritt A1) gebildeten Reaktionsgemishes mit wässriger Alkalihydroxidlösung, wobei eine wässrige Alkalichlorid-haltige Lösung und eine organische Phase erhalten wird, die das Gemisch der Di- und Polyamine enthält,
A3) Abtrennung der organischen Phase aus Schritt A2) von der wässrigen Phase und Aufarbeitung der organischen Phase zur Gewinnung des Gemischs der Di- und Polyamine der Diphenylmethanreihe,
A4) Einstellung der gemäß Schritt A3) verbliebenen, Alkalichlorid-haltigen Lösung auf einen pH-Wert < 8, Reinigung der Alkalichlorid-haltigen Lösung so, dass diese auf einen TOC-Wert < 200 ppm, bevorzugt < 50 ppm, ganz besonders bevorzugt < 20 ppm gebracht wird, wobei die Alkalichlorid-haltige Lösung ggf. zumindest teilweise mit den in Schritt A1) aus dem Reaktionsgemisch entfernten und /oder mit den bei der Aufarbeitung der organischen Phase gemäß Schritt A3) anfallenden wässrigen Phasen vereinigt wird,
B) Umsetzung des in Schritt A3) erhaltenen Gemischs der Di- und Polyamine der Diphenylmethanreihe mit Phosgen zu einem Gemisch von Di- und Polyisocyanaten der Diphenylmethanreihe (MDI) und Chlorwasserstoff,
**dadurch gekennzeichnet, dass**
C) die in Schritt A4) erhaltene gereinigte Alkalichlorid-haltige Lösung zumindest teilweise einer elektrochemischen Oxidation unter Bildung von Chlor, Alkalilauge und ggf. Wasserstoff zugeführt wird, und
D) wenigstens ein Teil des gemäß Schritt C) hergestellten Chlors zur Herstellung des in Schritt B) eingesetzten Phosgens eingesetzt wird.

2. Verfahren gemäß Anspruch 1, **dadurch gekennzeichnet, dass** wenigstens ein Teil der gemäß Schritt C) hergestellten Alkalilauge in die Neutralisation gemäß Schritt A) zurückgeführt wird.

3. Verfahren gemäß einem der Ansprüche 1 bis 2, **dadurch gekennzeichnet, dass** wenigstens ein Teil des in Schritt B) anfallenden Chlorwasserstoffs in Salzsäure überführt und diese Salzsäure zumindest teilweise bei der Umsetzung A1) des Anilins mit Formaldehyd als Katalysator eingesetzt wird.

4. Verfahren gemäß einem der Ansprüche 1 bis 3, **dadurch gekennzeichnet, dass** die Alkalichloridkonzentration der Alkalichlorid-haltigen wässrigen Lösung, die aus Schritt A4) erhalten wird, 2 bis 24 Gew.-%, bevorzugt 5 bis 15 Gew.-%, beträgt.

5. Verfahren gemäß einem der Ansprüche 1 bis 4, **dadurch gekennzeichnet, dass** die Alkalichlorid-haltige wässrige Lösung vor ihrem Einsatz in einer elektrochemischen Oxidation durch Wasserentzug und /oder durch Zugabe von Alkalichloriden in fester Form oder in Form konzentrierter Lösung auf einen Alkalichloridgehalt > 5 Gew.-%, bevorzugt > 10 Gew.-%, ganz besonders bevorzugt > 20 Gew.-% gebracht wird.

6. Verfahren nach Anspruch einem der Ansprüche 1 bis 5, **dadurch gekennzeichnet, dass** die Anilin/Formaldehyd-Umsetzung mit einem Protonierungsgrad von < 30 %, bevorzugt <20 %, besonders bevorzugt < 15 % und ganz besonders bevorzugt < 10 % durchgeführt wird.

7. Verfahren gemäß einem der Ansprüche 1 bis 6, **dadurch gekennzeichnet, dass** die elektrochemische Oxidation gemäß Schritt C) nach einer Alkali-Chlorid-Elektrolyse unter Einsatz von Ionenaustauschermembranen und/oder unter Einsatz von Gasdiffusionselektroden als Kathode erfolgt.

8. Verfahren nach Anspruch 7, **dadurch gekennzeichnet, dass** die Alkali-Chlorid-Elektrolyse nach dem Membranelektrolyseverfahren unter Einsatz einer Ionenaustauschermembran durchgeführt wird, wobei das Alkalichlorid der Alkalichlorid-haltigen Lösung Natriumchlorid ist und die Ionenaustauschermembran eine Wassertransportkapazität von > 3 mol Wasser / mol Natrium, bevorzugt > 4 mol Wasser / mol Natrium, besonders bevorzugt > 5 mol Wasser / mol Natrium aufweist.

9. Verfahren gemäß Anspruch 7 oder 8, **dadurch gekennzeichnet, dass** die der Alkalichlorid - Elektrolyse zugeführten Alkalichlorid-Lösungen vor der Elektrolyse auf einen pH-Wert < 7, bevorzugt pH < 6 eingestellt sind.

10. Verfahren gemäß einem der Ansprüche 7 bis 9, **dadurch gekennzeichnet, dass** die Alkalchlorid-konzentration der aus der Elektrolysezelle gelangenden Alkalichlorid-haltigen Lösung 100 bis 280 g/l, bevorzugt 110 bis 220 g/l beträgt.

11. Verfahren nach einem der Ansprüche 7 bis 10, **dadurch gekennzeichnet, dass** die Konzentration der aus der Elektrolyse erhaltenen Alkalilauge 13 bis 33 Gew.-%, bevorzugt 20 bis 30 Gew.-% beträgt.

## Claims

1. Process for preparing methylenediphenyl diisocyanates, which comprises at least the steps
A1) reaction of aniline with formaldehyde in the presence of hydrochloric acid to give a reaction mixture containing diamines and polyamines of the diphenylmethane series ("MDA"), if appropriate at least partially with removal of the water introduced with the starting materials or formed in the reaction from the reaction mixture,
A2) neutralization of the reaction mixture formed in step A1) using aqueous alkali metal hydroxide solution to give an aqueous solution containing alkali metal chloride and an organic phase containing the mixture of diamines and polyamines,
A3) separating off the organic phase from step A2) from the aqueous phase and working up the organic phase to give the mixture of diamines and polyamines of the diphenylmethane series,
A4) bringing the solution containing alkali metal chloride which remains after step A3) to a pH of < 8, purification of the solution containing alkali metal chloride so that it is brought to a TOC value of < 200 ppm, preferably < 50 ppm, very particularly preferably < 20 ppm, where the solution containing alkali metal chloride is, if appropriate, at least partly combined with the aqueous phases removed from the reaction mixture in step A1) and/or with the aqueous phases obtained in the work-up of the organic phase in step A3),
B) reaction of the mixture of diamines and polyamines of the diphenylmethane series obtained in step A3) with phosgene to give a mixture of diisocyanates and polyisocyanates of the diphenylmethane series (MDI) and hydrogen chloride,
**characterized in that**
C) the purified solution containing alkali metal chloride obtained in step A4) is at least partly fed to an electrochemical oxidation to form chlorine, alkali metal hydroxide and optionally hydrogen and
D) at least part of the chlorine produced in step C) is used for preparing the phosgene used in step B).

2. Process according to Claim 1, **characterized in that** at least part of the alkali metal hydroxide prepared in step C) is recirculated to the neutralization in step A).

3. Process according to either of Claims 1 and 2, **characterized in that** at least part of the hydrogen chloride obtained in step B) is converted into hydrochloric acid and this hydrochloric acid is at least partly used as catalyst in the reaction A1) of aniline with formaldehyde.

4. Process according to any of Claims 1 to 3, **characterized in that** the alkali metal chloride concentration of the aqueous solution containing alkali metal chloride which is obtained from step A4) is from 2 to 24% by weight, preferably from 5 to 15% by weight.

5. Process according to any of Claims 1 to 4, **characterized in that** the aqueous solution containing alkali metal chloride is brought to an alkali metal chloride content of > 5% by weight, preferably > 10% by weight, very particularly preferably > 20% by weight, by removal of water and/or by addition of alkali metal chlorides in solid form or in the form of a concentrated solution before being used in an electrochemical oxidation.

6. Process according to any of Claims 1 to 5, **characterized in that** the aniline/formaldehyde reaction is carried out at a degree of protonation of < 30%, preferably < 20%, particularly preferably < 15% and very particularly preferably < 10%.

7. Process according to any of Claims 1 to 6, **characterized in that** the elecktrochemical oxidation in step C) is carried out as an alkali metal chloride electrolysis using ion exchange membranes and/or using gas diffusion electrodes as cathode.

8. Process according to Claim 7, **characterized in that** the alkali metal chloride electrolysis is carried out by the membrane electrolysis process using an ion exchange membrane, with the alkali metal chloride of the solution containing alkali metal chloride being sodium chloride and the ion exchange membrane having a water transport capacity of > 3 mol of water/mol of sodium, preferably > 4 mol of water/mol of sodium, particularly preferably > 5 mol of water/mol of sodium.

9. Process according to Claim 7 or 8, **characterized in that** the alkali metal chloride solutions fed to the alkali metal chloride electrolysis are brought to a pH of < 7, preferably pH < 6, before the electrolysis.

10. Process according to any of Claims 7 to 9, **characterized in that** the alkali metal chloride concentration of the solution containing alkali metal chloride which leaves the electrolysis cell is from 100 to 280 g/l, preferably from 110 to 220 g/l.

11. Process according to any of Claims 7 to 10, **characterized in that** the concentration of the alkali metal hydroxide solution obtained from the electrolysis is from 13 to 33% by weight, preferably from 20 to 30% by weight.

## Revendications

1. Procédé pour la préparation de méthylène-diphényl-diisocyanates, au moins comprenant les étapes
A1) mise en réaction d'aniline avec du formaldéhyde en présence d'acide chlorhydrique, pour l'obtention d'un mélange réactionnel contenant des di- et polyamines de la série du diphénylméthane (« MDA »), éventuellement au moins en partie avec élimination à partir du mélange réactionnel de l'eau introduite avec les produits de départ ou formée dans la réaction,
A2) neutralisation du mélange réactionnel formé selon l'étape A1), à l'aide d'une solution aqueuse d'un hydroxyde de métal alcalin, avec obtention d'une solution aqueuse contenant un chlorure de métal alcalin et d'une phase organique qui contient le mélange des di- et polyamines,
A3) séparation de la phase organique provenant de l'étape A2) d'avec la phase aqueuse et traitement final de la phase organique pour l'obtention du mélange des di- et polyamines de la série du diphénylméthane,
A4) ajustement à un pH < 8 de la solution contenant un chlorure de métal alcalin, restant selon l'étape A3), purification de la solution contenant un chlorure de métal alcalin, de sorte que celle-ci est portée à une valeur COT < 200 ppm, de préférence < 50 ppm, de façon tout particulièrement préférée < 20 ppm, la solution contenant un chlorure de métal alcalin étant éventuellement au moins en partie réunie avec les phases aqueuses séparées du mélange réactionnel dans l'étape A1) et/ou avec les phases aqueuses produites lors du traitement final de la phase organique selon l'étape A3),
B) mise en réaction du mélange des di- et polyamines de la série du diphénylméthane, obtenu dans l'étape A3), avec du phosgène, pour l'obtention d'un mélange de di- et polyisocyanates de la série du diphénylméthane (MDI) et de chlorure d'hydrogène,
**caractérisé en ce que**
C) la solution contenant un chlorure de métal alcalin, purifiée, obtenue dans l'étape A4) est au moins en partie envoyée à une oxydation électrochimique avec formation de chlore, d'une solution d'hydroxyde de métal alcalin et éventuellement d'hydrogène, et
D) on utilise au moins une partie du chlore produit selon l'étape C) pour la production du phosgène utilisé dans l'étape B).

2. Procédé selon la revendication 1, **caractérisé en ce qu'**au moins une partie de la solution d'hydroxyde de métal alcalin, produite selon l'étape C), est renvoyée dans la neutralisation selon l'étape A).

3. Procédé selon la revendication 1 ou 2, **caractérisé en ce qu'**on convertit en acide chlorhydrique au moins une partie du chlorure d'hydrogène formé dans l'étape B) et on utilise cet acide chlorhydrique au moins en partie dans la réaction A1) de l'aniline avec du formaldéhyde en tant que catalyseur.

4. Procédé selon l'une quelconque des revendications 1 à 3, **caractérisé en ce que** la teneur en chlorure de métal alcalin de la solution aqueuse contenant un chlorure de métal alcalin, qui est obtenue à partir de l'étape A4), vaut de 2 à 24 % en poids, de préférence de 5 à 15 % en poids.

5. Procédé selon l'une quelconque des revendications 1 à 4, **caractérisé en ce qu'**avant son utilisation dans une oxydation électrochimique on porte la solution aqueuse contenant un chlorure de métal alcalin, par déshydratation et/ou par addition de chlorures de métaux alcalins sous forme solide ou sous forme de solution concentrée, à une teneur en chlorure en chlorure de métal alcalin > 5 % en poids, de préférence > 10 % en poids, de façon tout particulièrement préférée > 20 % en poids.

6. Procédé selon l'une quelconque des revendications 1 à 5, **caractérisé en ce que** la réaction aniline/formaldéhyde est effectuée à un degré de protonation de < 30 %, de préférence < 20 %, de façon particulièrement préférée < 15 % et de façon tout particulièrement préférée < 10 %.

7. Procédé selon l'une quelconque des revendications 1 à 6, **caractérisé en ce que** l'oxydation électrochimique selon l'étape C) s'effectue selon une électrolyse de chlorure de métal alcalin avec utilisation de membranes échangeuses d'ions et/ou avec utilisation d'électrodes à diffusion de gaz en tant que cathode.

8. Procédé selon la revendication 7, **caractérisé en ce qu'**on effectue l'électrolyse de chlorure de métal alcalin selon le procédé d'électrolyse sur membrane en utilisant une membrane échangeuse d'ions, le chlorure de métal alcalin de la solution contenant un chlorure de métal alcalin étant le chlorure de sodium et la membrane échangeuse d'ions présentant une capacité de transport d'eau de > 3 moles d'eau / mole de sodium, de préférence > 4 moles d'eau / mole de sodium, de façon particulièrement préférée > 5 moles d'eau / mole de sodium.

9. Procédé selon la revendication 7 ou 8, **caractérisé en ce qu'**avant l'électrolyse on ajuste à un pH < 7, de préférence pH < 6 les solutions de chlorure de métal alcalin envoyées à l'électrolyse de chlorure de métal alcalin.

10. Procédé selon l'une quelconque des revendications 7 à 9, **caractérisé en ce que** la teneur en chlorure de métal alcalin de la solution contenant un chlorure de métal alcalin arrivant de la cellule électrolytique vaut de 100 à 280 g/l, de préférence de 110 à 220 g/l.

11. Procédé selon l'une quelconque des revendications 7 à 10, **caractérisé en ce que** la concentration de la solution d'hydroxyde de métal alcalin obtenue à partir de l'électrolyse vaut de 13 à 33 % en poids, de préférence de 20 à 30 % en poids.
